# EUROPEAN PATENT APPLICATION

(11) **EP 4 312 223 A1**
(43) Date of publication of application: **31.01.2024**
(21) Application number: 22187872.1
(22) Date of filing: 29.07.2022
(51) Int. Cl.: G16H 10/60, G16H 40/63

(54) **PERSONALISED TREATMENT SOFTWARE**

(71) Applicant: Closed Loop Medicine Ltd, Cambridge CB22 3AT (GB)
(72) Inventor: Siddle, James Matthew, London, N8 7NA (GB); Fisher, Brett Mark, Guildford, GU1 4PB (GB)
(74) Representative: Potter Clarkson

(57) **Abstract**

A computer implemented method for providing a personalised therapeutic instruction comprising initialising a treatment program wrapper in a software environment; initialising a treatment program using the treatment program wrapper, wherein the treatment program wrapper provides runtime execution context to the treatment program and isolates the treatment program from the software environment; executing a personalised therapeutic process of the treatment program to output personalised therapeutic data; routing the personalised therapeutic data to a clinician portal using a clinician interface mechanism of the treatment program wrapper; and outputting the personalised therapeutic instruction to a user interface based on the personalised therapeutic data.

## Description

### Field

The present disclosure relates to a method and apparatus for providing a personalised therapeutic instruction.

### Background

Automated, personalised medicine programs have the potential to improve patient health outcomes for chronic diseases by increasing treatment feedback frequency and making automated or semi-automated therapeutic decisions based on regularly captured patient data.

There is a need to provide assurance that treatment decisions made by such an automated program are safe and reliable. The disclosed method and apparatus may improve the safety and/or reliability of automated personalised medicine software.

### Summary

According to a first aspect of the present disclosure there is provided a computer implemented method for providing a personalised therapeutic instruction comprising:
initialising a treatment program wrapper in a software environment;
initialising a treatment program using the treatment program wrapper, wherein the treatment program wrapper provides runtime execution context to the treatment program and isolates (processes of) the treatment program from the software environment;
executing a personalised therapeutic process of the treatment program to output personalised therapeutic data;
routing the personalised therapeutic data to a clinician portal using a clinician interface mechanism of the treatment program wrapper; and
outputting the personalised therapeutic instruction to a user interface based on the personalised therapeutic data.

The steps of initialising the treatment program, executing the personalised therapeutic process, routing the personalised therapeutic data and outputting the personalised therapeutic instruction may be performed by the treatment program wrapper.

The method may further comprise receiving the personalised therapeutic instruction from the clinician interface mechanism.

The personalised therapeutic data may comprise the personalised therapeutic instruction.

The method may comprise routing the personalised therapeutic instruction to the clinician interface mechanism of the computer program wrapper.

The method may further comprise receiving an approved personalised therapeutic instruction from the clinician interface mechanism. Outputting the personalised therapeutic instruction may comprise outputting the approved personalised therapeutic instruction.

The method may comprise awaiting receipt of an approval of, or a revision to, the personalised therapeutic instruction, from the clinician interface mechanism.

The approved personalised therapeutic instruction may comprise an approval of the personalised therapeutic instruction or a clinician update to the personalised therapeutic instruction.

The wrapper may isolate the treatment program by controlling the flow of input data and output data to and from the treatment program respectively using one or more input interfaces and one or more output interfaces.

The clinician interface mechanism may include at least one of the one or more input interfaces and at least one of the one or more output interfaces.

The wrapper may provide runtime execution context by configuring an underlying device, virtualised environment, platform, operating system and/or the software environment.

The method may further comprise:
comparing a performance of the underlying device, virtualised environment, platform, operating system and/or the software environment against requirements of a service level agreement; and
generating a warning, failing to initialise the treatment program and/or terminating the treatment program if the requirements of the service level agreement are not satisfied.

The method may further comprise:
initiating and/or monitoring workflow events using the treatment program wrapper; and
communicating a detected workflow event to the treatment program, wherein executing the process of the treatment program comprises executing the process of the treatment program in response to the detected workflow event.

The workflow event comprises one or more of:
a patient or clinician completing a task or failing to complete a task;
an event time being reached;
a patient accepting, rejecting or requesting a deferral to a personalised therapeutic instruction;
a patient requesting a clinician consultation;
receipt of input data;
a change in automation state;
a patient being offline for a threshold time; and
a fault report.

The method may comprise retrieving a time reference using the treatment program wrapper. The method may comprise initiating and/or monitoring the workflow events using the time reference; and/or providing the time reference to the treatment program.

Retrieving the time reference may comprise:
retrieving the time reference from a backend server;
retrieving the time reference from an underlying operating system;
retrieving the time reference from a bios clock; or
retrieving the time reference from a network time protocol.

The method may further comprise:
changing an automation state of the treatment program using the wrapper, in response to one or more of:
receiving a request to change the automation state from the clinician interface mechanism;
receiving a request to change the automation state from the treatment program; and
an event time being reached.

The method may further comprise:
validating, with the treatment program wrapper, input data from an input data source against one or more expected values; and
executing the process of the treatment program using the input data if the validation is successful.

If the validation is unsuccessful, the method may further comprise:
generating a log message;
initiating an adverse event workflow; or
terminating the treatment program and preventing further execution.

The one or more expected values may be predefined.

The method may comprises receiving one or more expected values via a programmable plug-in mechanism such that the validating step is configurable for the treatment program.

The method may comprise:
performing one or more safety checks on the personalised therapeutic instruction; and
outputting the personalised therapeutic instruction to the user interface if the personalised therapeutic instruction satisfies the one or more safety checks; or
performing one or more safety actions if the personalised therapeutic instruction does not satisfy the one or more safety checks.

The one or more safety actions may comprise any of:
generating and outputting a warning;
stopping the treatment; and
initiating a call to the emergency services

The personalised therapeutic instruction may comprise one or more of:
an instruction to change a therapy;
an instruction to change a dosage amount and/or a dosage timing of a therapy;
an instruction to undertake or change a level or particular activity of cognitive behavioural therapy;
provision of a new or updated prescription;
an instruction to suspend or terminate a treatment;
an instruction to commence, or change a frequency of, physiological measurements; and
an instruction to seek immediate medical attention.

The wrapper and the treatment program may form a container image for a container engine.

According to a second aspect of the present disclosure there is provided an apparatus comprising:
one or more processors; and
memory storing instructions that when executed by the one or more processors, cause the one or more processors to perform any method disclosed herein.

According to a third aspect of the present disclosure there is provided a non-transitory, computer-readable storage medium storing instructions that when executed by one or more processors, cause the one or more processors to perform any method disclosed herein.

There may be provided a computer program, which when run on a computer, causes the computer to configure any apparatus, including a circuit, controller, converter, or device disclosed herein or perform any method disclosed herein. The computer program may be a software implementation, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software may be an assembly program.

The computer program may be provided on a computer readable medium, which may be a physical computer readable medium such as a disc or a memory device, or may be embodied as a transient signal. Such a transient signal may be a network download, including an internet download. There may be provided one or more non-transitory computer-readable storage media storing computer-executable instructions that, when executed by a computing system, causes the computing system to perform any method disclosed herein.

### Brief Description of the Drawings

One or more embodiments will now be described by way of example only with reference to the accompanying drawings in which:
Figure 1 illustrates a treatment application for providing a personalised therapeutic instruction according to an embodiment of the present disclosure;
Figure 2 illustrates a system for running the treatment application of Figure 1;
Figure 3 illustrates a method of providing a personalised therapeutic instruction according to an embodiment of the present disclosure; and
Figure 4 a specific example method that may be performed by a treatment wrapper executing a process of a treatment program for insomnia.

### Detailed Description

Automated, personalised medicine programs have the potential to improve patient health outcomes for Chronic diseases by increasing treatment feedback frequency and making automated or semi-automated therapeutic decisions based on regularly captured patient data.

There is a need to provide assurance that treatment decisions made by such an automated program are safe, reliable, consistently audited, and utilise patient data in line with their wishes.

Specifically, there is a need to provide treatment decisions that are:
a) appropriately isolated to prevent unwanted interactions with other processes that could prevent or impede correct performance of the medical device software,
b) based on validated data from trusted sources,
c) made with respect to a consistent and reliable date/time frame of reference,
d) made in a timely manner according to the requirements of the treatment protocol,
e) have a clear lifecycle that is aligned with the treatment protocol, from treatment initiation through to completion,
f) allow for clinician "on-the-loop" and "in-the-loop" workflows, where aspects of decisions are made directly by humans (e.g. setting a dose based on collected data)
g) subject to safety checks before being committed and then shared with patients,
h) audited consistently in line with regulatory requirements.

It is also important to moderate any use of externally evaluated machine-learned models to ensure that the performance of automated treatment programs is consistent, and satisfies underlying medical device requirements.

The disclosed method and apparatus can address one or more of the above issues by providing a treatment program wrapper. The treatment program wrapper can provide a managed execution environment that provides the resources and system characteristics that a treatment program needs to operate correctly, safely, and in line with key medical device and healthcare regulations.

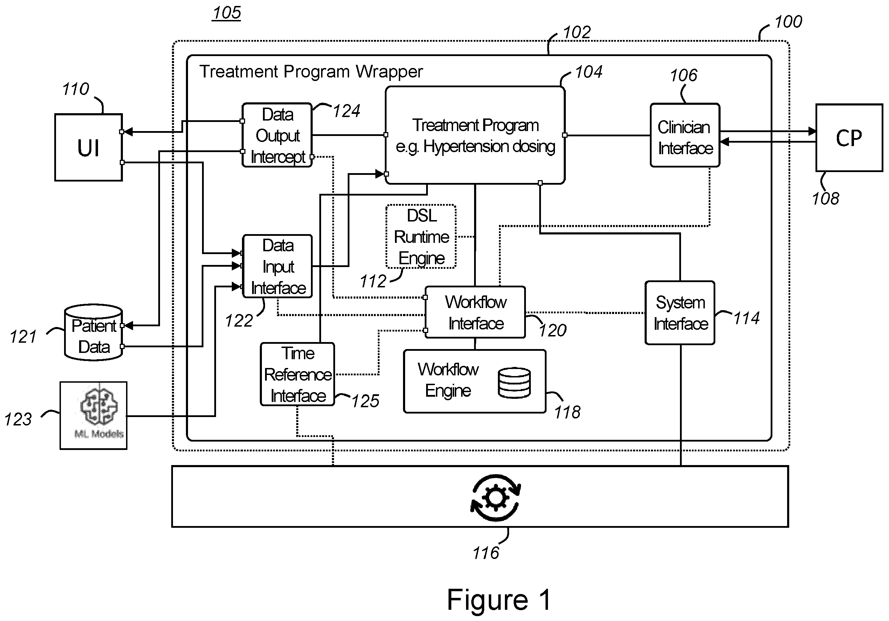

Figure 1 illustrates a treatment application 100 for providing a personalised therapeutic instruction according to an embodiment of the present disclosure. The treatment application 100 comprises a treatment program wrapper 102 and a treatment program 104.

The treatment program wrapper 102 (which may be referred to herein as the wrapper 102) provides runtime execution context to the treatment program 104 and isolates processes of the treatment program 104 from a software environment 105 in which the treatment application 100 resides. The wrapper 102 may include a workflow engine 118 which can manage execution of personalised therapeutic processes of the treatment program 104 to provide personalised therapeutic data. In this example, the wrapper 102 includes a clinician interface mechanism 106 for outputting the personalised therapeutic data to a clinician portal 108. In some examples, the personalised therapeutic data may comprise the personalised therapeutic instruction, which may be reviewed, updated and/or approved by a clinician using the clinician portal 108. In other examples, the clinician may provide the personalised therapeutic instruction to the clinician portal 108 based on the personalised therapeutic data and the wrapper 102 may receive the personalised therapeutic instruction from the clinician portal 108 via the clinician interface mechanism 106. In both cases, the wrapper 102 outputs the personalised therapeutic instruction to a user interface (UI) 110 based on the personalised therapeutic data.

The wrapper 102 advantageously provides isolation and execution context to the treatment program 104 such that the treatment program 104 runs correctly and safely. In addition, by routing personalised therapeutic data output by the treatment program 104 to the clinician interface 106, qualified clinicians can ensure that the personalised treatment data and/or instructions are adequately supervised and/or acted upon.

As disclosed herein, a "personalised therapeutic instruction" comprises an instruction to for a patient on how to conduct a treatment therapy. Example instructions include, changing a drug dosage amount or dosage timing or frequency (which may be referred to herein as drug titration), undertaking or increasing a level or particular activity of cognitive behavioural therapy, instructing, or changing a frequency of, physiological measurements, seeking immediate medical attention, the provision of a new or updated prescription etc. A personalised therapeutic instruction may also be referred to as a personalised therapeutic recommendation in that a patient may choose to reject (or accept) the instruction and feedback the rejection (or acceptance) to the treatment program 104 which may subsequently forward the rejection to the clinician portal 108.

As disclosed herein, a "treatment program wrapper 102" comprises a computer program wrapper that provides an isolated environment for the execution of a personalised medicine treatment program 104. The treatment program wrapper 102 provides an interface between the treatment program 104 and the underlying software environment 105 such that direct communication between the treatment program 105 and the software environment 105 (and any other programs or tasks running in the software environment 105) is prevented (isolation). The wrapper 102 may be provided as a set of interfaces for interfacing the treatment program 104 to the underlying software environment 105.

As disclosed herein the "software environment 105" comprises all computer implemented methods, other than those of the treatment program wrapper 102 and the treatment program 104, running on one or more processors (and/or other computer hardware) that are executing the computer implemented methods of the wrapper 102 and the treatment program 104. Example computer implemented methods that the treatment program 104 is isolated from includes those defining: the operating system operation, hardware interfaces (e.g. network communication, user interface protocols etc.), other software programs, firmware, virtualised computing environments, partitioned computing environments, container engines etc.

As disclosed herein, "runtime execution context" comprises predefined program execution points such as functions or methods that the treatment program 104 implements. The predefined program execution points can provide a predictable, constrained means for the treatment program 104 to receive control. The functions may include call-back functions that provide the treatment program 104 with a means of interacting with the wrapper 102, and thus with the wider software environment 105 and the outside world. The runtime execution context may also comprise an associated runtime state that the treatment program 104 can access to understand the parameters of the execution context. The runtime execution context may be provided by one or more of the interfaces described below.

As explained in greater detail below, the wrapper 102 isolates the treatment program 104 from the underlying software environment 105, such as the operating system 116 or other software programs. The isolation may be provided by one or more input and/or output interfaces of the wrapper 102, such as the clinician interface mechanism 106. The wrapper 102 also provides runtime execution context to the treatment program 104. The wrapper 102 may configure and define all interactions of the application 100 with the underlying software environment, operating system 116, hardware (including the UI 110 and the clinician portal 108) and/or outside world and present a consistent and reliable execution context to the treatment program 106. As described below, the wrapper 102 can also control lifecycle management of the treatment program 104. The wrapper 102 may be defined by a core software library that can be compiled together with code defining the treatment program 104, to define the treatment application 100 comprising the wrapper 102 and the treatment program 104. In view of the isolating, context provision and software library features, the wrapper 102 can provide a template construct of a treatment application for use with different treatment programs 104. In this way, developers of treatment programs 104 can concentrate on the logic and coding requirements required for the corresponding treatment and trust the core software library and resulting wrapper 102 to provide guarantees with respect to execution context, safe and reliable operation through isolation, and lifecycle management. The wrapper 102 may be considered to be the core package described in US non-provisional application 63/145077 which is hereby incorporated by reference in its entirety.

As described herein, a treatment program 104 refers to software code or a software program that processes patient data to provide personalised therapeutic data for a patient. In this way, the treatment program 104 can perform at least a portion of a treatment of the patient. The treatment program 104 may be considered to be software as a medical device (SAMD). In some examples, the treatment program 104 may process patient data (such as daily blood pressure readings) to provide personalised therapeutic data (such as a statistical summary of the daily blood pressure readings). In some examples, the treatment program 104 may process patient data and make treatment decisions resulting in personalised therapeutic instructions (such as a change in dose regimen of a prescribed drug). In some examples, the treatment program 104 may be a Turing-complete program written in a general purpose programming language, such as Java, JavaScript, Swift, C++ etc. In some examples, the program may be written in a Personalised Medicine Domain Specific Language (DSL) as described in US non-provisional application 63/255711 which is hereby incorporated by reference in its entirety. The DSL can constrain the set of programs that can be expressed and executed to a predefined subset with predictable safety characteristics.

### Isolation

The treatment program wrapper 102 provides the runtime execution context for the treatment program and provides the necessary isolation to ensure correct performance of the SAMD. In examples with a DSL treatment program 104, the wrapper can include a DSL runtime engine 112 for ensuring safe automation of the DSL treatment program 104.

The wrapper 102 can isolate the treatment program and provide the runtime execution context through a set of interfaces. One such interface is the clinician interface mechanism 106. The set of interfaces can ensure that all interactions between the treatment program 104 and other processes or tasks (including UI 110 interactions) are mediated and managed. The set of interfaces can be application level interfaces. The set of interfaces can control all information flow to and from the treatment program 104. The set of interfaces may be provided by the core software library. The treatment program 104 can interact with the set of interfaces to handle lifecycle events, read data, propagate decisions etc. Unless otherwise specified, the functionality of the treatment program wrapper 102 with respect to the treatment program 104, as described herein, is provided through one or more interfaces of the set of interfaces.

As described herein, an interface may comprise one or more functions or methods that can: (i) provide events and/or objects to the treatment program 104 (for example, a workflow interface 120 (see below) may provide a new measurement event to the treatment program); (ii) call methods of the treatment program 104 (for example, the wrapper 102 can call a personalised therapeutic process of the treatment program 104); and/or (ii) receive requests or calls from the treatment program 104 and optionally provide an object, event or program state in return. The treatment program 104 may include complementary hooks (functions or methods) to receive input from, or make calls to, the interfaces of the wrapper.

In some examples, the set of interfaces may extend to the operating system level and the treatment program wrapper 102 and treatment program 104 may together form a container image for operation on a Docker (RTM) engine or similar virtualisation technology. This can advantageously further increase the level of isolation (and safety) provided for the treatment program 104.

Isolating the treatment program 104 through the set of interfaces advantageously improves the safety of the provision of personalised therapeutic instructions, by avoiding unexpected interference from external sources. In some examples, the level and type of isolation imposed may depend on the safety classification and architecture of the SAMD, where there may or may not be a risk of other processes interacting in unexpected / unwanted ways with the treatment program 104 or a need to prevent the treatment program 104 from attempting to bypass the capabilities provided by the wrapper 102.

Although Figure 1 illustrates a number of interfaces, it will be appreciated that the described interfaces are not exhaustive and the wrapper may provide other interfaces.

### Runtime Execution

The treatment program wrapper 102 may include a system interface 114 that interfaces with the underlying operating system 116 to provide the runtime execution context to the treatment program 104. The system interface 114 can configure processes, threads, tasks and timers provided by the operating system 116 (or an underlying platform between the operating system 116 and application, such as a runtime environment). In this way, the system interface 114 can ensure that the wrapper 102 can act on behalf of the treatment program 104 to provide runtime execution context. As a result, the treatment program 104 can respond to events such as time passing or user interaction at the UI 110, in a timely manner. By configuring processes of the operating system 116, the system interface 114 can also support workflow management (via the workflow interface 120), as described in more detail below.

The wrapper 102 or system interface 114 may comprise a wrapper service level agreement (wrapper SLA) for defining a required or desired level of responsiveness. A specific implementation of the wrapper 102 may be rated to satisfy certain SLAs based on the underlying mobile device, platform and/or operating system 116. The wrapper 102 or system interface 114 may generate a warning, fail to initialise the application 100 or terminate the application 100 if the SLA cannot be met due to platform limitations.

The system interface 114 and/or SLA may be time dependent. For example, the system interface 114 may configure the operating system 116 for particular time periods at which processes of the treatment program 104 are scheduled to execute.

In a specific example, a treatment program 104 may require runtime execution context once per day, as close to 7pm as possible, but no later than 10 minutes after, to check if a clinician has adjusted a drug dosage. The system interface 114 can configure the underlying operating system 116 or mobile platform with a timer and an associated priority level that means sufficient runtime execution context will be provided to the container on time. For example, the system interface 114 may instruct the operating system 116 or platform to throttle or stop other non-essential processes (e.g. games, messaging application etc). The wrapper 102 may request permissions from the operating system 116 or platform at installation to ensure that the system interface 114 can perform such configuration. If the operating system configuration is in line with the SLA, the application 100 initialises successfully.

Although the treatment program 104 may be related to chronic conditions where a timeliness may be measured in minutes, days or weeks rather than second or milliseconds, there may be certain safety critical scenarios where a rapid response to events is required (for example, patient blood pressure readings indicate immediate medical attention is required). Therefore, the chronic nature of the treatments should not imply speed is not required.

### Treatment Lifecycle Management

A treatment of the treatment program 104 has required steps such as a start, an end and numerous steps in between to successfully treat a patient. As a result, processes of the treatment program 104 should execute to provide treatment updates, interventions, reports etc at different stages of a treatment in a way that is aligned with a treatment protocol.

The wrapper 102 may include a workflow engine 118 to provide lifecycle management of the treatment delivered by the treatment program 104. The workflow engine 118 can support delivery of the treatment through a standard series of events. The workflow engine 118 may include and coordinate a schedule of events. The workflow engine 118 can communicate, trigger or execute the events through a workflow interface 120 of the wrapper 102. The workflow interface 120 can provide an interface between the workflow engine 118 and the treatment program 104 to communicate an event to or from the treatment program 104 and/or trigger, invoke or execute processes of the treatment program 104. The treatment program 104 may include a complementary hook or interface for connecting to the workflow interface 120 to receive event triggers, process execution instructions, output event reports, initiate a change of program state etc.

For a DSL treatment program 104, the workflow interface 120 can interface with the DSL runtime engine 112 and trigger execution of a process of the treatment program 104 via the DSL runtime engine 112.

The workflow interface 120 may also interface with the system interface 120 to receive system level parameters, for example a time reference or an indication that the SLA is satisfied. The workflow engine 118 can trigger execution of a process of the treatment program 104 based on the schedule of events and the system level parameters. The workflow engine 120 may also communicate with the system interface 120 to indicate upcoming events that require a particular runtime execution context. The system interface 114 may configure the operating system 116 in line with the indicated runtime execution context.

The functionality of the workflow engine 118 may include one or more of:
µ Providing external integration points (e.g. via the schedule of events) that allow the wrapper 102 to detect workflow-significant events and pass these on (as triggers, execution instructions etc)to the treatment program 104 via the workflow interface 120;
• Providing the workflow interface 120 of the workflow engine 118 to interface with the corresponding interface of the treatment program 104 through which all workflow events can be communicated;
• Supporting long running processes, along with both synchronous and asynchronous interaction models;
• Enabling gating on treatment stages, along with other standard workflow capabilities such as branching pathways;
• Managing workflow state on behalf of the treatment program 104, allowing the treatment program 104 to query current workflow state as well as initiate state changes.

Examples of workflow events include:
- Initial treatment lifecycle events:
   ∘ Patient has installed the treatment application onto their device;
   ∘ Patient has completed treatment onboarding / training;
   ∘ Patient has configured their regular routine;
   ∘ Patient has completed a baselining period of data collection;
- Ongoing treatment events include:
   ∘ A scheduled daily check-in time has arrived;
   ∘ Patient has provided a new biometric reading;
   ∘ Patient has failed to complete a required activity;
   ∘ Patient accepts, rejects or requests deferral to a personalised therapeutic instruction;
   ∘ Patient requests a clinician consultation, optionally following receipt of personalised therapeutic instruction;
   ∘ A scheduled notification has been generated and viewed by the patient;
   ∘ A change in automation state;
   ∘ A detected fault;
   ∘ A clinician interaction such as:
      ▪ Generating, updating or approving a personalised therapeutic instruction;
      ▪ Initiating a review;
- End-of-treatment events include:
   ∘ A scheduled treatment off-boarding time has arrived;
   ∘ Patient has decided to stop the treatment;

In some examples, the workflow engine 118 can offer an application programming interface (API) for the treatment program 104 to initiate significant changes in treatment, which the workflow engine 118 can then take responsibility for actioning. Example state changes include:
- Changing the current phase of the treatment from "baselining" to "active titration." For example, the treatment program 104 moves from a phase of baselining the patients response to an initial drug prescription to actively instructing the patient to take a new drug dosage (with clinician approval or at least clinician informing).
- Changing a level of automation or clinician participation e.g. from "in the loop" to "on the loop" as described below.
- Registering a treatment decision so it can be acted on by the wider treatment application 100.
- Storing derived data for later use by the treatment program 104 or the wider treatment application 100.
- Reporting an adverse event.

State changes may be initiated in a number of ways. For example, a process of the treatment program 104 may make a treatment decision that outputs a state change. The state change may be communicated to the clinician interface mechanism 106 for review and optionally approval by a clinician. The treatment program 104 may communicate the state change to the workflow engine 118 via the workflow interface 120 and the workflow engine 118 may update the state of the application 100. In some examples, a clinician may instruct a state change via the clinician portal 108 and clinician interface mechanism 106. The treatment program 104 may receive the state change and communicate this to the workflow engine 118. In some examples, the clinician interface mechanism 106 may interact with the workflow interface 120 via the workflow interface 120, such that the workflow engine 118 can receive the clinician state change instruction directly from the clinician interface mechanism 106. In some examples, the workflow engine 118 may independently update the state in response to a particular time being reached. For example, the workflow engine 118 may update the state from baselining to active titration when two weeks have elapsed since baselining commenced. Such a state change may be communicated to the clinician portal 106, via the workflow interface 120 and clinician interface mechanism 106 and optionally via the treatment program 104, for clinician approval.

The wrapper 102 or workflow engine 118 can ensure that workflow events passed to the treatment program 104 conform to the SLAs described previously. The lifecycle/event management provided by the workflow engine 118 advantageously allows the treatment program 104 to focus on treatment decision making logic rather than managing difficult time handling concerns, for example. Failure scenarios are handled through the well-defined workflow interface 120, providing a degree of separation from the treatment logic of the treatment program 104.

It will be appreciated that although some interfaces of the wrapper 102 are illustrated and described as providing a direct interface between the treatment program 102 and another entity such as the operating system 116, UI 110 or clinician portal 108, any of the interfaces disclosed herein may be controlled or co-ordinated by the workflow engine 118 via the workflow interface 120, as indicated by the broken lines between the workflow interface 120 and the other interfaces in Figure 1. In some examples, one or more of the clinician interface mechanism 106, the system interface 114, the data input interface 122, or the data output interface 124 may form part of the workflow interface 120. In some examples, the workflow engine 118 and workflow interface 120 can provide a single master interface to the treatment program 104 and co-ordinate onward communication with external entities such as the operating system 116, UI 110 or clinician portal 108 via the one or more corresponding external interfaces. In other words, all the clinician interface mechanism 106, the system interface 114, the data input interface 122, and the data output interface 124 communicate with the treatment program 104 via the workflow interface 120 and do not communicate directly with the treatment program 104.

### Clinician Interface Mechanism

The clinician interface mechanism 106 provides a clinician with sufficient opportunity to interact with and direct any automated treatments. For example, the clinician interface mechanism 106 can allow a clinician to set or approve a new drug dosage (amount and/or time/frequency). The clinician interface mechanism 106 may prompt a clinician to make a decision during the course of a patient's treatment.

Two particular examples include:
1. **Clinician is "in the loop"** - The clinician plays an active role in decision making such as reviewing recently captured data and selecting a new dose, or approving a new dose that has been recommended by the automated treatment. In other words, the clinician makes or approves treatment decisions. In such an example, the treatment program 104 outputs personalised therapeutic data to the clinician portal 108 via the clinician interface mechanism 106. In some examples, the clinician may review the personalised therapeutic data and provide a personalised therapeutic instruction (treatment decision) in response. In some examples, the personalised therapeutic data may include a personalised therapeutic instruction for review and approval at the clinician portal. In any "in the loop" example, the wrapper 102 or treatment program 104 awaits input from the clinician portal 108 via the clinician interface mechanism 106, in the form of an approved personalised therapeutic instruction, before proceeding to implement the instruction.
2. **Clinician is "on the loop"** - The clinician is kept informed of automated decisions but is not required to play an active role. In this example, the treatment program 104 provides the personalised therapeutic data to the clinician interface mechanism 106 in the same way as described above. However, clinician intervention is optional and would typically be passive if the treatment is going well. Therefore, the treatment program 104 may proceed to implement an automated personalised therapeutic instruction without waiting for approval from the clinician interface mechanism 106.

In both examples, the wrapper 102 can provide for the clinician interaction through the workflow engine 118 described above. In particular, the workflow engine 118 can manage required communications to and from the clinician interface mechanism 106 using long-running transactions and asynchronous interactions, communicated via the workflow interface 120.

As a specific example for insomnia, the workflow engine 118 may notify treatment program 104 that two weeks have passed since a previous dosage change. In response, the treatment program 104 may retrieve the last two weeks of sleep diary records and calculate a new time for the patient to take Melatonin before going to bed. In some examples, the treatment program 104 can flag that human intervention is needed to approve the decision. The treatment program 104 can pass control back to the wrapper to obtain the approval. Specifically, the wrapper 102 outputs the dosage change to the clinician interface mechanism 106 (optionally via the workflow engine 118 and workflow interface 120) for clinician approval. In some examples, the clinician interface mechanism 106 may query an automation state (also referred to as a clinician participation state) of the treatment program/application. If the automation state indicates that clinician approval (in the loop) is required for dosage changes, the clinician interface will await clinician approval of, or revision to, the new dosage instruction. If the automation state indicates that clinician approval is not required for dosage changes (on the loop) the wrapper can implement the instruction as described below. For in the loop, the wrapper 102 can receive an approved personalised therapeutic instruction (updated or approved) from the clinician portal 108 via the clinician interface mechanism. Once the approved instruction is received, the wrapper 102 can invoke the treatment program 104 (optionally via the workflow engine 118 and workflow interface 120) to pass on / implement the approved instruction, for example by updating a routine of the patient to set the new time for taking Melatonin. The new routine may be communicated to the UI 110, optionally via the workflow engine 118 and interface 120 and optionally following further safety checks as described below.

The disclosed wrapper 102 architecture and the clinician interface mechanism can work together synergistically to advantageously support varying degrees of automation. For example, the wrapper architecture can support all stages of an inform, drive, automate protocol. In an initial phase or a baselining phase, the treatment application 100 may support an inform protocol in which the application simply provides a mediation mechanism to pass information between a user (patient) and a clinician. For example, the user may provide patient data such as daily blood pressure measurements. The treatment program 104 may process the daily measurements and output personalised therapeutic data, such as a statistical summary of the daily measurements over a two week period. The wrapper 102 may pass the data to the clinician interface mechanism 106 for review. An automation state (set to "inform" or equivalent) indicates to the clinician interface mechanism 106 that it should await a personalised therapeutic instruction from the clinician (e.g. maintain dosage, change dosage, discontinue treatment etc.). The application 100 can then handle the personalised therapeutic instruction and convey it to the user interface 110.

In the drive mode, the application 100 operates in a semi-automated mode (e.g. when the baselining phase is complete and the treatment program 104 has sufficient data to make personalised therapeutic decisions). The application 100 may operate the same as in the inform mode, except that the treatment program will output a "suggested" personalised therapeutic instruction. The automation level may be set to "drive" indicating that the clinician interface mechanism 106 should await an approval of, or revision to, the personalised therapeutic instruction, from the clinician portal 108. In this way, the application 100 begins to "drive" the treatment but is still subject to clinician participation in the decision making.

In the automate mode, the application 100 operates in a fully automated mode (e.g. when the patient is showing an improving or stable response to the treatment). The application 100 may operate the same as in the drive mode, except that the clinician interface mechanism 106 is only required to inform the clinician and does not await approval. The automation level may be set to "automate" indicating that the clinician interface mechanism 106 does not need to await an approval of, or revision to the personalised therapeutic instruction, from the clinician portal 108.

### Providing Trusted Data Sources

Processes of the treatment program 104 may output treatment decisions or instructions based on data inputs from one or more potential data sources 121 with different associated timelines. Example data can include patient biometric information (e.g. blood pressure readings), demographic data (e.g. weight or age) or population-derived datasets such as BMI tables or indicators of adverse event risk for different patient groups. Data sources may comprise local and/or remote data sources 121 (such as memory storage, databases (drug database), online resources, (regulatory requirements, clinical guidelines etc), electronic health records, prescription services and other healthcare IT systems), and/or user input data received via the UI 110, patient measurements received via a connected medical device (e.g. blood pressure monitor). Regardless of data source, the wrapper 102 may provide a data input interface 122 to ensure that input data for the treatment program 104 is: a) validated; and b) from a trusted data source. The data input interface 122 can provide an access mechanism to pre-registered data sources and perform schema-based validation checks against data retrieved from these data sources. In this way, the wrapper 102 can advantageously act as a mediator in ensuring the validity and trustworthiness of data used for decision making.

User input data received via the UI 110 may include: an acceptance or rejection of a personalised therapeutic instruction with an optional request for clinician follow-up; a request that a personalised therapeutic instruction be deferred; or user input of physiological data, symptom data, well-being data etc.

The treatment program 104 may (only) retrieve any required data from data sources via the wrapper 102 / data input interface 122. The data input interface 122 may provide the sole access mechanism to the associated data via a runtime reference (or object). In some examples, the treatment program 104 may query the runtime reference. In some examples, the runtime reference may output events for consumption by the treatment program 104. Whether the runtime reference is queried or outputs events may depend on the nature of the data source. In either case, all data passing through the data input interface 122 to the treatment program 104 is checked against a predefined schema (of expected values) for correctness.

As a specific example, the data input interface 122 may provide a runtime reference for a blood pressure reading data source. The runtime reference may emit "new BP reading" events. The events may conform to a well-defined schema of entities, data fields, and types (also referred to herein as expected values). The data input interface 122 may validate each reading to detect and flag (or reject) implausible values. Validation of such a reading could extend to checking the relationship between a between systolic and diastolic readings.

The data input interface 122 may register input data sources with the treatment wrapper 102 during initialisation. The input data may be annotated with meta-data that provides information on the integrity, nature, source, provenance, and/or timestamp of the associated data. During operation, the data input interface 122 may check the annotations against expected values to provide a level of assurance that the data source is acceptable for use in the treatment.

As a specific example, the data input interface 122 may register a data source for a connected Blood Pressure device. The data input interface 122 may refer to a list of medical devices that can safely be used with the encapsulated treatment (in other words expected values of the device make and model). During treatment, the patient may connect a blood pressure measurement device that conforms to the data retrieval protocol but is not on the list of safe devices. In response, the data input interface 122 identifies that the connected device is not on the annotated list and may prevent input of the data to the treatment program and/ or prevent the treatment program 104 from running.

As a further specific example, the data input interface 122 may register a data source to provide a table of Blood Pressure readings with an associated risk of adverse events. The data source may be annotated with type classification, unique identifier, and date which together denote the provenance of the data (e.g. POPULATION RISK RATINGS, MHRA STANDARD BP AE RISK LEVELS, 2017). During initialisation, the data input interface 122 checks these annotations against a list of acceptable sources of risk data, finds that the data source is no longer acceptable, and either prevents the treatment from starting or flags a warning for consideration by an operational team.

The data input interface may check the integrity of input data by checking the hashing of the data object (e.g. MD5, SHA-256, etc) to ensure the object has not been corrupted or manipulated since the origin.

In some examples, the set of allowed input data sources may be locked. For example, the set of data sources may be managed by an independent group or body. This can ensure that the treatment program 104 would be prevented from defining its own data sources and thus data provenance and access would be tightly controlled. The wrapper 102 may update the set of allowed input data sources in response to receiving an update from the independent group or body, receiving a new configuration (see below) or via an update to one of the allowed input data sources, for example a regulated resource library or database. The update from the independent body may be provided using APIs for example, third party APIs from the independent body.

In some examples, the data input interface 122 may perform checks against predefined expected values. In some examples, the one or more expected values may be defined through a programmable plug-in mechanism and the set of specific checks performed may be configurable for each personalised treatment program 104. The data input interface 122 may receive a treatment configuration as an input (see below) with a set of personalised expected values. The data input interface 122 may check the personalised expected values are within a general range of predefined values.

In some examples, data input interface 122 may check data source annotations to apply patient GDPR preferences. For example, the data input interface may prevent certain patient data from being read and used as part of their treatment. In this case, the wrapper 102 may prevent the treatment program 104 from running. Alternatively, the treatment program 104 may account for certain patient data being unavailable.

In some examples, the input data sources may include externally hosted machine learned models 123, rules based algorithms, and/or decision trees. In other words, the approach described here for the use of trusted data sources can be extended to cover the use of externally hosted models and algorithms. In such examples, the set of annotations and checks applied may differ, but the core principle remains the same.

In some examples, the treatment program 104 and/or the machine learned models 123 and other may have an element of configurability. For example, for a Hypertension treatment program, such configurability could include the setting of expected blood pressure changes, adverse event severities, baseline period, minimum adherence threshold for dosing decisions etc, for a particular patient. In some examples, such configuration may be provided as a data input source and handled by the data input interface 125. The data input interface 125 may check and validate a configuration file, for example, to ensure expected values are within predefined general bounds, that the configuration file has been approved or regulated, or that the configuration file was authored or amended by a trusted signatory. In examples implementing DSL treatment programs 104, the configuration may be provided and validated as part of the DSL program.

### Time Source Reference

SAMDs that automate therapeutic decisions can be dependent on the reliable reporting of time to perform correctly. Although superficially straightforward, accurate time provision can be nuanced and challenging.

For example, different mobile digital devices can have their own understanding of when "now" is, and while this will typically be retrieved from a time server synchronized to a robust and reliable source (such as the US Naval Observatory), patients or other actors may adjust the local time reported on a given device.

Inaccurate time provision can be further exacerbated on mobile devices by network availability issues, daylight saving changes, and in particular the time-zone where the device is located. In extreme cases, relativity may come into play.

As an example, a patient travelling internationally by aeroplane may disable their network in one country, receive treatment events from a treatment program while flying, turn their network back on and suddenly their device will be reporting a different time. As a result, treatment decisions made on the basis of time reported by the mobile device may be invalid and unsafe without careful risk mitigation.

The treatment program wrapper 102 can address the issue of accurate time provision by providing a single source of truth for "now" to the treatment program 104 running within its context. The single source of time truth may be provided by a time reference interface 125 of the wrapper 102. The time reference interface 125 may also provide a supporting library of functions (accessible to the treatment program 104) that allow the treatment program to reliably process and compare dates, times, and especially durations.

The time reference interface 125 may maintain a consistent time derived from: a local time of a backend server known to have a reliable clock; a lower level operating system clock (as illustrated) that cannot be modified by users; and/or application of a network time protocol. In some examples, the time reference interface 125 may form part of the workflow interface 120 or the system interface 114. The treatment program 104 can request the current date and time from the time reference interface 125 at any time. The treatment program 104 and/or the workflow engine 118 may manage the treatment orchestration events (treatment lifecycle and workflow events) based on the single source of true time provided by the time reference interface 125. As a result, the treatment program 104 can advantageously focus on therapeutic logic rather than challenging time reference issues.

### Safety Checks

A closed-loop automated therapeutic SAMD has the potential to cause patient harm and will typically be classified at the highest level. A lack of human involvement increases the potential for a malfunctioning system to run unimpeded and unnoticed, significantly increasing the potential harm to patients for many types of medical device software.

Many of the capabilities of the treatment program wrapper 102 described above already contribute to system safety by:
- Isolating the treatment program 104 from external interference;
- Making sure the treatment program 104 is using validated or correct data or machine-learned models for decision making;
- Providing a reliable clock to use for all treatment decisions;
- Providing a runtime execution context for ensuring the treatment program 104 can act in time to meet the needs of the protocol; and
- Providing a standard workflow engine 118 for chronic disease treatment.
- Providing the DSL runtime engine 112 for executing DSL treatment programs 104 defined according to a proprietary grammar for precision medicine, restricting the set of treatments to a limited subset with predictable safety characteristics.

In addition the wrapper 102 can improve patient safety by providing a data output intercept 124 through which all outputs of the treatment program 104 must pass. The data output intercept is a plug-in interceptor mechanism and can provide a way for a variety of different safety checks to be applied, and for interventions to be applied if appropriate.

The wrapper 102 may configure the data output intercept 124 to apply a set of safety checks to outputs of the treatment program 104. The safety checks may apply primarily to data storage events and treatment decisions (personalised therapeutic instructions), but may extend to workflow state changes and other scenarios. In other words, any output initiated by the treatment program 104 may be passed through the interceptor mechanism. Figure 1 illustrates a sole data output intercept 125 intercepting outputs for data storage in local or remote memory 121 and treatment outputs (e.g. personalised therapeutic instructions) to the UI 110. However, in other examples, the data output intercept 125 may also capture further outputs of the treatment program 104, such as the personalised therapeutic data provided to the clinician interface mechanism 106 and/or any output provided to the workflow interface 120, such as state changes or event reports.

The output data intercept 124 provides a means of processing all outputs from the treatment program 104, and allows a broad range of checks to be applied - from data validation to detection of adverse events. The set of safety checks may include one or more predefined, mandatory safety filters, such as checking a dosage amount and/or frequency against a regulated reference. The set of safety checks may include one or more configurable safety checks that may vary according to application requirements.

The output data intercept 124 may apply different sets of filters depending on the nature of the output. For data storage events, the output data intercept 124 may validate data, for example, by checking or detecting anomalous values of biometric data fields. For events or outputs of the treatment program 104 (such as a personalised therapeutic instruction) that actively modify the patient's treatment, e.g. setting a new dose, the output data intercept 124 may check the personalised therapeutic instruction against patient risk factors (e.g. by referring to a drug database resource or demographic data) such as the potential for causing an adverse event.

If the output data intercept 124 detects a potential safety concern, the intercept 124 take action in a number of ways. For example, the output data intercept 124 may generate a warning for review by a clinician (via the clinician interface), produce an Adverse Event Report, stop the treatment, initiate a call to the emergency services etc. The action may be co-ordinated and/or performed by the workflow engine 118 and the workflow interface 120.

The output data intercept 124 monitors automated decision making logic of the treatment program 104. The output data intercept 124 can provide a set of standard, independent safeguards that are applied to any action or influence of the treatment program 104, thereby preventing or reducing the risk of harm to patients and ultimately improving overall patient outcomes.

### Audit decisions in line with regulatory requirements

The wrapper 102 may include an audit mechanism for all interactions with the treatment program 104. The audit mechanism may take the form of a log of inflows and outflows through the strict boundary of the wrapper 102, along with a timestamp derived from the time reference interface 125. Together, these provide an audit trail covering the data and events that prompted the treatment program 104 to act, and any resulting down-stream outputs or actions from the program 104. The audit log can be stored locally alongside the wrapper 102, though when used on a mobile device with limited storage the log will typically be sent to a server-based backend for long term storage. In this scenario, a local data-retention policy can be applied by the wrapper to ensure that critical audit log data was retained in the event of a mobile device being unable to connect to the server for a long period of time.

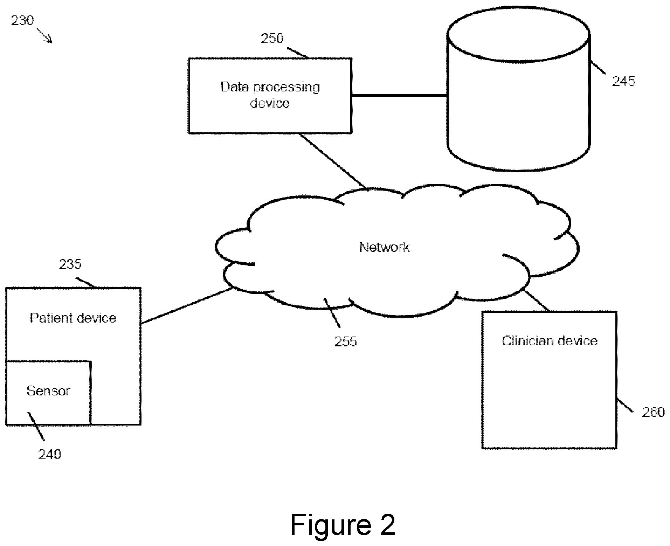

The disclosed treatment application 100, treatment program wrapper 102 and treatment program 104 may be installed on a stand-alone computing system or distributed across a plurality of computing systems. The computer implemented methods of the treatment application 100, treatment program wrapper 102 and treatment program 104 may stored on a memory and executed using one or more processors. A system 230 suitable for running the application 100 and performing any of the computer implemented methods disclosed herein is shown in Figure 2.

System 230 includes a patient device 235. In some examples, the patient device 235 may comprise one or more processors configured to perform any of the methods disclosed herein. However, in this example, the patient device 235 only serves to provide the UI 110 and a plurality of data input sources via: (i) user input via the UI 110; and (ii) sensor readings from sensor 240.

In the illustrated embodiment, patient device 235 is a smartphone, optionally comprising the sensor 240. However, the invention is not limited in this respect and the patient device 235 can take many other forms, including but not limited to a mobile telephone, a tablet computer, a desktop computer, a voice-activated computing system, a laptop, a gaming system, a vehicular computing system, a wearable device, a smart watch, a smart television, an internet of things device and a medication-dispensing device.

The patient device 235 may act as a data input source by gathering data, including patient data, relating to a patient and/or the immediate environment. Patient device 235 may gather patient data from the sensor 240, which can be any combination of: a light sensor such as a camera, a temperature sensor, an acoustic sensor such as a microphone, an accelerometer, an air pressure sensor, an airborne particulate sensor, a global positioning sensor, a humidity sensor, an electric field sensor, a magnetic field sensor, a moisture sensor, an air quality sensor and a Geiger counter, an electroencephalogram (EEG), a heart rate monitor, a pulse oximeter, or any other sensor capable of gathering polysomnographic relevant data and/or any other characteristic of the patient and/or the patient's immediate environment. Although, the sensor 240 is illustrated as forming part of the patient device 235, in some embodiments the sensor 240 may be separate to, and remotely communicate with, the patient device 235. In other examples, sensor 240 can be omitted from patient device 235.

Alternatively, or in addition, information about the patient and/or the immediate environment of the patient can be obtained via other mechanisms including manual data entry using the UI 110 of the patient device 235.

The patient device 235 may comprise a memory (not illustrated) for storing patient data. Such data may also be stored on a database 245 as networked or cloud-based data storage.

The patient device 235 may have one or more sub-applications installed on a storage medium associated with the patient device (not shown). The one or more sub-applications may provide a front end of the treatment application 100 and connect the UI 110 and data input source to the treatment application 100 hosted on a processing device 250 on a back-end server. The one or more sub-applications may be configured to control data acquisition via sensor 120 and/or to assist the patient in providing data relating to their current condition and/or their immediate environment. The one or more sub-applications may be downloaded from a network, for example from a website or an online application store. Installation of the sub-application may trigger installation of the full treatment application 100 on the system 230.

In this example, the system 230 further comprises the data processing device 250 hosted (or installed) on a server and communicatively coupled to the patient device 235 via a network 255. In the illustrated embodiment network 255 is the internet, but the invention is not limited in this respect and network 255 could be any network that enables communication between patient device 235 and data processing device 250, such as a cellular network or a combination of the internet and a cellular network.

The data processing device 250 may include a memory for hosting the treatment application 100 and one or more processors for performing any of the computer implemented methods disclosed herein. In other words, the data processing device 250 may host: the treatment program wrapper 102, including the set of interfaces, and perform all of the functionality of the wrapper disclosed herein; and the treatment program 104 and perform all of the functionality of the treatment program 104 disclosed herein. For example, in some embodiments, the data processing device 250 may initialise the wrapper 102 in a software environment of the back-end server and initialise the treatment program 104 using the wrapper 102. In some examples, the wrapper 102 and the treatment program 104 may form a container image. The data processing device 250 may comprise a container engine for running the container image. The wrapper 102, operating on the data processing device 250, may execute the personalised therapeutic process of the treatment program 104 and route personalised therapeutic data to a clinician device 260 using the clinician interface mechanism 106 of the wrapper 102. The wrapper 102 can then output the personalised therapeutic instruction to the user interface 110 of the patient device 235 (via a data output intercept 125). In this way, the data processing device 250 provides networked, server based or cloud based, processing capability to the system 230 for performing the disclosed computer implemented methods.

The data processing device 250 may be coupled to the database 245 that can store patient data and/or provide a data input source, as described earlier in the application.

The clinician device 260 provides the clinician portal 108 described above and enables a clinician to provide "on the loop" or "in the loop" participation. The clinician device 260 is communicatively coupled via network 255 to the data processing device 250. Clinician device 260 may be physically located at a clinician's premises during its use, such as a doctor's surgery, a pharmacy or any other healthcare institution, e.g. a hospital or may comprise a mobile device such as a tablet or smartphone.

It is also contemplated that clinician device 260 is typically used by a medically trained person with appropriate data security clearance, such that advanced functionality may be available. For example, the clinician device 260 may be able to access a medical history of the patient, generate a prescription for the patient, place an order for medication, etc. Access to functionality may be controlled by a security policy implemented by a local processor.

In the same way as described above for the patient device 235, the clinician device 260 may comprise a sub-application installed that is compatible with the treatment application 100 installed on the data processing device 250.

It will be appreciated that the various steps of the computer implemented methods disclosed herein may be performed in any combination by any of the one or more processors in the patient device 235, the data processing device 250 and the clinician device 260. For example, as described above, all steps may be performed by the data processing device 250 on a back-end server with the patient device 235 merely providing the UI 110 and a data input source and the clinician device 260 providing the clinician portal 108. In a further example, the data processing device 250 may be omitted and all steps may be performed on the patient device 235, with a direct network connection to the clinician device 260 acting as the clinician portal 108. In a yet further example, functionality of the treatment application 100 may be distributed between the data processing device 250 and the patient device 235.

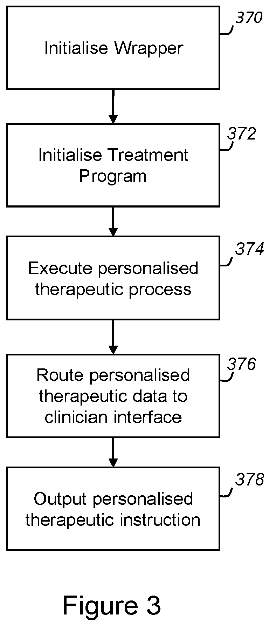

Figure 3 illustrates a method of providing a personalised therapeutic instruction according to an embodiment of the present disclosure. The method may be performed upon execution of the treatment application 100, for example on data processing device 250.

A first step 370 of the method comprises initialising a treatment program wrapper in a software environment. A second step 372 comprises initialising a treatment program using the treatment program wrapper 102. The treatment program wrapper 102 provides runtime execution context to the treatment program and isolates processes of the treatment program 104 from the software environment 105. A third step 374 comprises executing a personalised therapeutic process of the treatment program 104 to output personalised therapeutic data. A fourth step 376 comprises routing the personalised therapeutic data to a clinician portal 108 using a clinician interface mechanism 106 of the treatment program wrapper 102. A fifth step 378 comprises outputting the personalised therapeutic instruction to a user interface 110 based on the personalised therapeutic data.

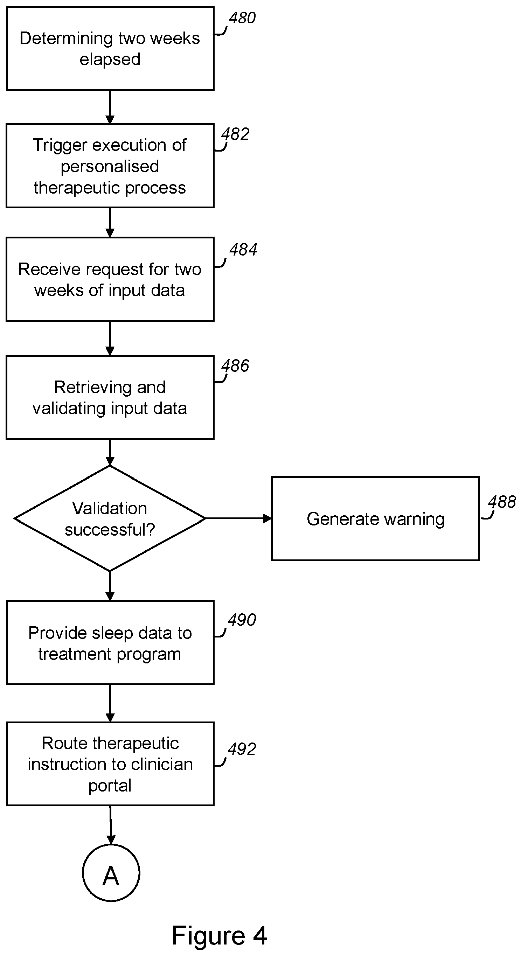

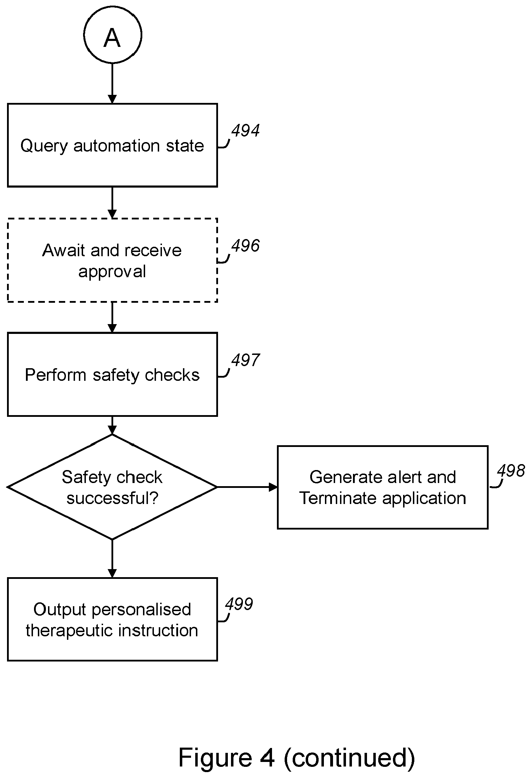

Figure 4 illustrates a specific example method that may be performed by the treatment wrapper 102 executing a process of a treatment program 104 for insomnia.

Following initialisation of the wrapper 102 and treatment program 104, a first step 480 may comprise the workflow engine 118 determining that two weeks have passed since a previous dosage change. The workflow engine 118 may determine that two weeks have elapsed by receiving a time reference from the time reference interface 125.

A second step 482 comprises the workflow engine 118 triggering execution of a personalised therapeutic process of the treatment program 104 via the workflow interface 120. The workflow interface 120 may trigger execution by invoking the process or by notifying the treatment program 104 of the event that two weeks have elapsed since the last dosage change. In other words, the relevant logic (or portions thereof) for mapping the two week elapse to invoking the process may be handled by the treatment program 104 or the workflow engine 118.

A third step 484 may comprise the wrapper 102 receiving a request from the treatment program 104 to retrieve the last two weeks of sleep diary records from a relevant data input source, e.g. local memory 121. The wrapper 102 may receive the request at the data input interface 122, optionally via the workflow interface 120.

A fourth step 486 comprises retrieving the two weeks of sleep data and validating the sleep data using the data input interface 122. A decision point determines if the validation was successful. If the validation was not successful, the method proceeds to step 488 and terminates the application and outputs an error. If validation is successful, the method proceeds to step 490 and provides the sleep data to the treatment program 104.

Execution of the personalised therapeutic process proceeds and outputs personalised therapeutic data comprising a personalised therapeutic instruction comprising a new dosage time for the patient to take Melatonin before going to bed.

The method proceeds to step 492 in which the wrapper 102 receives the treatment instruction (new dosage) from the treatment program 104 and routes the instruction to the clinician portal 108 using the clinician interface mechanism 106. At step 494, the clinician interface mechanism 106 may query the automation state from the workflow engine 118 (via the workflow interface 120). The workflow engine 118 may indicate that the automation state is or is not at a level that a dosage change requires clinician approval (in the loop). The method proceeds to optional step 492, if clinician in the loop is indicated, and the clinician interface mechanism 106 awaits and receives clinician approval or a revised instruction.

Optionally (not shown), the wrapper 102 may return the approved therapeutic instruction to the treatment program 104 for further processing. For example, the treatment program 104 may output the updated dose to the workflow engine 118 via the workflow interface.

The method continues to step 497 and the data output intercept 125 receives the (approved) therapeutic instruction (from the treatment program 104 or directly from the clinician interface mechanism 106) and performs safety checks on the instruction. A decision point determines if the personalised therapeutic instruction passes the safety checks. If the safety checks are not met, the method proceeds to step 498 and takes remedial action such as generating a warning and terminating the application 100. If the safety checks are successfully passed, the method proceeds to step 499 and outputs the new dosage to the user interface 110 for the patient.

Other examples that illustrate the capability of the interfaces of the wrapper to provide isolation, runtime execution and lifecycle management include:
1. The workflow engine 118 may receive an indication from the time reference interface 125 that a threshold time period has elapsed. The workflow engine may initiate a review of the patient's treatment by providing a relevant event to, or calling a method of, the treatment program 104 using the workflow interface. In this way, the workflow interface (optionally working with the system interface 114) can provide runtime execution context to the treatment program, providing the treatment program 104 the opportunity to perform the review. The treatment program 104 may review historic readings, select a new dose, and then make a request via the clinician interface mechanism 106 for an interaction with a clinician to confirm the new dose. The example illustrates that the interfaces 120, 114, 106 isolate the treatment program 104 from the underlying software environment 105. In addition, the treatment program 104 lies idle until called by the workflow interface 120.
2. The data input interface 122, optionally in conjunction with the workflow interface 120, may notify the treatment program that a patient has entered a new biometric reading. The wrapper 102 can provide runtime execution context (via system interface and/or workflow interface 120) to the provide treatment program 104 the opportunity to process the new biometric data and - for example - make a request via the clinician interface mechanism 106 for a clinician intervention.
3. The data input interface 122, optionally in conjunction with the workflow interface 120, may notify the treatment program that a patient has accepted a new personalised therapeutic instruction but requested a clinician in person follow-up. The wrapper 102 can provide runtime execution context (via system interface and/or workflow interface 120) to provide the treatment program 104 the opportunity to gather relevant patient data and make a request via the clinician interface mechanism 106 for a clinician appointment.

It will be appreciated that any reference to "close to", "before", "shortly before", "after" "shortly after", "higher than", or "lower than", etc, can refer to the parameter in question being less than or greater than a threshold value, or between two threshold values, depending upon the context.

## Claims

1. A computer implemented method for providing a personalised therapeutic instruction comprising:
initialising a treatment program wrapper in a software environment;
initialising a treatment program using the treatment program wrapper, wherein the treatment program wrapper provides runtime execution context to the treatment program and isolates the treatment program from the software environment;
executing a personalised therapeutic process of the treatment program to output personalised therapeutic data;
routing the personalised therapeutic data to a clinician portal using a clinician interface mechanism of the treatment program wrapper; and
outputting the personalised therapeutic instruction to a user interface based on the personalised therapeutic data.

2. The method of claim 1, wherein the steps of initialising the treatment program, executing the personalised therapeutic process, routing the personalised therapeutic data and outputting the personalised therapeutic instruction are performed by the treatment program wrapper.

3. The method of claim 1 or claim 2, wherein the method further comprises:
receiving the personalised therapeutic instruction from the clinician interface mechanism.

4. The method of any preceding claim, wherein the personalised therapeutic data comprises the personalised therapeutic instruction.

5. The method of claim 4, wherein the method comprises:
routing the personalised therapeutic instruction to the clinician interface mechanism of the computer program wrapper.

6. The method of claim 5, further comprising:
receiving an approved personalised therapeutic instruction from the clinician interface mechanism,
wherein outputting the personalised therapeutic instruction comprises outputting the approved personalised therapeutic instruction.

7. The method of any preceding claim, wherein the wrapper isolates the treatment program by controlling the flow of input data and output data to and from the treatment program respectively using one or more input interfaces and one or more output interfaces.

8. The method of any preceding claim, wherein the wrapper provides runtime execution context by configuring an underlying device, virtualised environment, platform, operating system and/or the software environment.

9. The method of any preceding claim, further comprising:
initiating and/or monitoring workflow events using the treatment program wrapper; and
communicating a detected workflow event to the treatment program, wherein executing the process of the treatment program comprises executing the process of the treatment program in response to the detected workflow event.

10. The method of claim 9 comprising:
retrieving a time reference using the treatment program wrapper; and
initiating and/or monitoring the workflow events using the time reference; and/or
providing the time reference to the treatment program.

11. The method of any preceding claim, further comprising:
validating, with the treatment program wrapper, input data from an input data source against one or more expected values; and
executing the process of the treatment program using the input data if the validation is successful.

12. The method of any preceding claim comprising:
performing one or more safety checks on the personalised therapeutic instruction; and
outputting the personalised therapeutic instruction to the user interface if the personalised therapeutic instruction satisfies the one or more safety checks; or
performing one or more safety actions if the personalised therapeutic instruction does not satisfy the one or more safety checks.

13. The method of any preceding claim, wherein the personalised therapeutic instruction comprises:
an instruction to change a therapy;
an instruction to change a dosage amount and/or a dosage timing of a therapy;
an instruction to undertake or change a level or particular activity of cognitive behavioural therapy;
provision of a new or updated prescription;
an instruction to suspend or terminate a treatment;
an instruction to commence, or change a frequency of, physiological measurements; and
an instruction to seek immediate medical attention.

14. The method of any preceding claim wherein the wrapper and the treatment program form a container image for a container engine.

15. An apparatus comprising:
one or more processors; and
memory storing instructions that when executed by the one or more processors, cause the one or more processors to perform the method of any preceding claim.
